# EUROPÄISCHE PATENTSCHRIFT

(11) **EP 2 795 569 B1**
(45) Veröffentlichungstag und Bekanntmachung des Hinweises auf die Patenterteilung: **19.09.2018**
(21) Anmeldenummer: 13702438.6
(22) Anmeldetag: 30.01.2013
(51) Int. Cl.: G06Q 50/22

(54) **AUTHENTISIERUNGSSYSTEM FÜR MOBILE GERÄTE ZUM DATENAUSTAUSCH VON MEDIZINISCHEN DATEN**
SYSTEM FOR AUTHENTICATION OF MOBILE DEVICES FOR EXCHANGE OF MEDICAL DATA
SYSTÈME POUR L'AUTHENTICATION DES APPAREILS MOBILES POUR L'ÉCHANGE DES DONNÉES MEDICALES

(30) Priorität: 02.02.2012 DE 102012201505
(43) Veröffentlichungstag der Anmeldung: 29.10.2014
(73) Patentinhaber: Siemens Healthcare GmbH, 91052 Erlangen (DE)
(72) Erfinder: HAIDER, Sultan, 91052 Erlangen (DE); HEIDENREICH, Georg, 91056 Erlangen (DE)
(86) Internationale Anmeldenummer: PCT/EP2013/051799
(87) Internationale Veröffentlichungsnummer: WO 2013/113756

(56) Entgegenhaltungen:
- WO-A1-2011/011454
- US-A1- 2005 021 937
- US-A1- 2007 067 833
- US-A1- 2007 074 040
- US-A1- 2010 146 085
- KOUFI V ET AL: "HDGPortal: A Grid portal application for pervasive access to process-based healthcare systems", PERVASIVE COMPUTING TECHNOLOGIES FOR HEALTHCARE, 2008. PERVASIVEHEALTH 2008. SECOND INTERNATIONAL CONFERENCE ON, IEEE, PISCATAWAY, NJ, USA, 30. Januar 2008 (2008-01-30), Seiten 121-126, XP031289750, ISBN: 978-963-9799-15-8

## Beschreibung

Die vorliegende Erfindung liegt auf den Gebieten der Medizintechnik und der Informationstechnologie und betrifft ein Authentisierungssystem zur Authentisierung von mobilen, elektronischen Geräten gegenüber einem zentralen Server zum gesicherten Datenaustausch von medizinischen Daten.

Bei heutigen, modernen medizinischen Systemen, die in der überwiegenden Zahl von Kliniken verwendet werden, liegen die Daten als digitale Daten vor und werden auch über entsprechende Netzwerke in digitaler Form ausgetauscht. Im Gegensatz zu früheren Systemen, bei denen beispielsweise in der Radiologie noch Röntgenaufnahmen von Patienten in Form von Röntgenfolien abgelegt und gespeichert wurden, werden die heute erfassten radiologischen Bilddaten digital erfasst, weiterverarbeitet und/oder an andere (darunter auch externe Instanzen) weitergeleitet. Ein Vorteil der digitalen Datenverarbeitung in der Medizin ist in der sehr einfachen und flexiblen Weiterleitung und Verfügbarmachung der medizinischen Datensätze zu sehen. Der Datenaustausch basiert üblicherweise auf einem speziellen Protokoll, beispielsweise dem DICOM-Protokoll (DICOM: Digital Information and Communication in Medicine). Hat ein Patient die Klinik wieder verlassen, so ist es für ihn unter Umständen auch wichtig, auf seine medizinischen Bilddaten zugreifen zu können. Dies wird unter anderem dann wichtig, falls Nachsorgeuntersuchungen notwendig werden, für die die bereits erfassten Bilddaten oder anderweitige Patienten-Daten verfügbar sein müssen. Diese Daten liegen auf einem Klinik-Repository. Mit dem Transfer von digitalen Daten gehen jedoch auch Probleme einher. So ist es für Angreifer leichter, unauthorisierte Zugriffe auf diese Datensätze auszuführen. Mit anderen Worten ist es unerlässlich, die digitalen Daten vor unauthorisierten Zugriffen zu schützen und dabei dennoch eine leichte Zugänglichkeit der Daten für den Patienten zu ermöglichen. Dabei sollen auch die datentechnischen Ressourcen der beteiligten ComputerInstanzen mitberücksichtigt werden (Bandbreite, Adressierung von Gerätekomponenten etc.)

Identifikations- und Authentifizierungssysteme sind im Stand der Technik hinlänglich bekannt. Dabei werden in der Regel bekannte kryptologische Prozeduren eingesetzt, um die Identität eines Anwenders nachweisen zu können, um ihn gegenüber dem System zu authentisieren. Insbesondere in medizinischen Systemen ist es unabdingbar, dass sichere Kommunikationskanäle zur Kommunikation zwischen Patient und Klinik bzw. klinischen Datensätzen bereitgestellt werden können. Um einen möglichst flexiblen Zugang des jeweiligen Patienten auf seine klinischen Datensätze zu ermöglichen, ist es wünschenswert, mobile elektronische Geräte, wie z.B. Smartphones oder mobile Telekommunikationseinrichtungen zu verwenden, über die der Patient in der Regel ohnehin verfügt. Dafür ist es erforderlich, die persönlichen Zugangsdaten auf dem jeweiligen elektronischen Gerät zu speichern und über die bekannten standardisierten Kommunikationskanäle zu übertragen, wie z.B. den jeweiligen Mobilfunkbetreiber. Es liegt auf der Hand, dass bei einem solchen Datenaustausch die persönlichen Zugangsdaten sehr leicht beschädigt oder kompromittiert werden können. Die Mobilfunkverbindungen können relativ leicht durch einen Angreifer manipuliert werden, um die Zugangsdaten oder andere persönliche Daten abzugreifen. Deshalb sind zusätzliche Sicherungsmaßnahmen erforderlich, die jedoch mit einem hohen Verwaltungsaufwand verbunden sind, insbesondere auf der Seite des Patienten bzw. des mobilen Gerätes. Bekannte Systeme basieren deshalb auf der Bereitstellung einer Sicherungs-Infrastruktur, die in der Regel von Drittanbietern bereitgestellt wird, um für die mobilen Geräte verfügbar zu sein. Neben dem hohen Verwaltungsaufwand liegt ein weiterer Nachteil bei den bekannten Systemen im Stand der Technik darin, dass die Verfügbarkeit einer sicheren Authentisierung durch die Verfügbarkeit einer sicheren Authentisierung durch die Einrichtung spezifischer Sicherungs-Infrastrukturen verringert wird.

Die Druckschrift US 2007/067833 A1 betrifft ein System zum Ermöglichen von sicheren Transaktionen über ein Netzwerk, das eine Netzwerkanwendung zum Speichern von Sever-Client-Information und zum Aktivieren von Clients für sichere Transaktionen, einen Software-Treiber für eine Authentifizierung, der für den Netzwerkserver zugänglich ist, ein Software-Applet zum Erzeugen eines sicheren Datensatzes und zum Verwalten einer Übertragung und Aktualisierung des Inhalts des Datensatzes und eine Schallquelle in der Form einer Datei oder eine Schallerzeugungsvorrichtung umfasst.

Die Druckschrift US 2007/074040 betrifft ein Verfahren, ein System, ein mobiles Endgerät und ein Computerprogrammprodukt zum Authentifizieren der Signatur eines Benutzers. Das Authentifizierungsschema vereint die Vorteile der beiden biometrischen und digitalen Signaturverfahren durch die Projektion einer Folge von vordefinierten Bildern auf einer Oberfläche, die es dem Benutzer erlaubt, die projizierten Bilder zu unterschreiben, den Unterschreibevorgang in Form beispielsweise eines Video-Clips zu erfassen, die digitale Signatur des Benutzers auf den Clip seiner biometrischen Signatur anzuwenden und dann die biometrischen und digitalen Unterschriften zu verwenden, um den Benutzer zu authentifizieren.

Die Druckschrift US 2010/146085, betrifft einen Echtzeitkernel, der Echtzeitkommunikationen zwischen Kommunizierenden unterstützt, die auf den jeweiligen Knoten arbeiten.

Die vorliegende Erfindung hat deshalb die Aufgabe, die Identifikation und Authentisierung von Patienten über mobile elektronische Geräte gegenüber einem zentralen System, in dem medizinische Datensätze abgelegt sind, zu verbessern und zu vereinfachen, wobei gleichzeitig die Sicherheit sowohl für den Betreiber des mobilen Gerätes als auch für den Betreiber des zentralen Servers verbessert werden soll. Gleichzeitig werden Geräte-Komponenten modifiziert und in neuartiger Weise grundsätzlich abweichend adressiert, um die Sicherheit des Authentifizierungssystems steigern zu können.

Diese Aufgabe wird durch die beiliegenden, nebengeordneten Patentansprüche gelöst, insbesondere durch ein Authentisierungssystem, durch eine Instanziierungseinheit, durch ein Verfahren zur Authentisierung und durch ein Computerprogrammprodukt.

Nachstehend wird die Lösung der Aufgabe in Bezug auf das beanspruchte System beschrieben. Hierbei erwähnte Merkmale, Vorteile und/oder alternative Ausführungsformen sind ebenso auch auf die anderen beanspruchten Gegenstände (und somit auf die Instanziierungseinheit und auf das Computerprogrammprodukt und das Verfahren) zu übertragen und umgekehrt. Mit anderen Worten können die anderen Anspruchsformen auch mit den Merkmalen weitergebildet werden, die im Zusammenhang mit dem System beschrieben und/oder beansprucht sind. Dabei werden die entsprechenden funktionalen Merkmale des Systems oder des Verfahrens durch entsprechende gegenständliche Module ausgebildet, insbesondere durch Hardware-Module bzw. Mikroprozessorchip-Module, die auf dem elektronischen Gerät oder auf der Instanziierungseinheit implementiert sind. Grundsätzlich können die einzelnen Module bzw. Einheiten des Systems als Software-Module und/oder Mikroprozessorchip-Modul ausgebildet werden.

Gemäß einem Aspekt betrifft die Erfindung ein Authentisierungssystem zur Authentisierung jeweils eines mobilen, elektronischen Gerätes (wobei grundsätzlich eine Vielzahl von mobilen, elektronischen Geräten an das System angeschlossen ist und authentisiert werden soll) gegenüber einem zentralen Server zum gesicherten Datenaustausch von medizinischen Daten zwischen Gerät und Server, wobei der Server seinerseits in Datenaustausch steht mit einem klinischen System und Zugriff auf ein Repository mit klinischen bzw. medizinischen Daten (umfassend auch Patienten-Daten) hat, umfassend:
- Eine zentrale Instanziierungseinheit, die zur Instanziierung des jeweiligen Gerätes bestimmt ist, indem die Instanziierungseinheit jeweils eine individualisierte Patienten-spezifische bzw. User-spezifische Applikation als Verschlüsselungseinheit auf dem jeweiligen Gerät installiert und wobei die Applikation einen Schlüssel und eine Geräte-Kennung in versteckter Form in einem Programmspeicher des Gerätes ablegt bzw. installiert, wobei die Instanziierungseinheit eine Zuordnung zumindest zwischen Geräte-Kennung und Schlüssel in einem zentralen geschützten Speicher ablegt (auf den auch eine Entschlüsselungseinheit Zugriff hat, z.B. einer zentralen Registry).
- Eine Verschlüsselungseinheit, die lokal auf dem Gerät installiert ist und zum Erzeugen einer digitalen Signatur bestimmt ist, wobei die Signatur durch Verschlüsselung mit dem von der Instanziierungseinheit hinterlegten Schlüssel erzeugt wird, wobei die Signatur erzeugt wird aus einem Signatur-Urbild, das die Geräte-Kennung und einen Zeitstempel umfasst, und wobei die Verschlüsselungseinheit weiterhin zum Versenden zumindest der Signatur mit der Geräte-Kennung an den Server bestimmt ist.
- Eine Entschlüsselungseinheit, die auf dem zentralen Server installiert ist und wobei die Entschlüsselungseinheit ein Zugriffsmodul auf den zentralen, gesicherten Speicher umfasst und mittels des Zugriffsmoduls oder direkt auf diesen zentralen, gesicherten Speicher zugreifen kann, wobei die Entschlüsselungseinheit dazu bestimmt ist, die vom Gerät gesendete Signatur mit der Geräte-Kennung zu empfangen und aus der Geräte-Kennung unter Zugriff auf den zentralen, geschützten Speicher den jeweils zugeordneten Schlüssel zur Entschlüsselung auszulesen, um mit dem ausgelesenen Schlüssel die empfangene Signatur zu entschlüsseln und vom resultierenden Signatur-Urbild als Entschlüsselungsergebnis die Geräte-Kennung auszulesen, wobei die Entschlüsselungseinheit weiterhin dazu ausgebildet ist, das Entschlüsselungsergebnis mit der empfangenen Geräte-Kennung auf Übereinstimmung zu vergleichen und bei Übereinstimmung ist die Entschlüsselungseinheit weiter dazu bestimmt, mit der ausgelesenen Geräte-Kennung einen Zugriff auf das Repository auszuführen.

Im Folgenden werden die im Rahmen dieser Patentanmeldung verwendeten Begrifflichkeiten näher definiert und erläutert.

Grundsätzlich dient das Authentisierungssystem zum Nachweis der Identität des Patienten, der sein mobiles, elektronisches Gerät, insbesondere sein Smartphone, bedient gegenüber einer Registry, die als Server implementiert sein kann und die in Datenaustausch mit einem klinischen Repository steht.

Das mobile, elektronische Gerät ist vorzugsweise ein Handy oder ein Smartphone oder ein sonstiges elektronisches Gerät, das nicht ortsgebunden ist und insbesondere über ein Mobilfunknetz in Datenaustausch mit anderen Instanzen steht. Alternativ kann es sich auch um ein anderes Datenübertragungsnetzwerk, z.B. ein Funknetz oder das Internet, handeln. Das jeweilige elektronische Gerät ist einem Patienten zugeordnet und fungiert als Client des zentralen Servers und umfasst zumindest einen Datenspeicher und einen Programmspeicher oder einen geschützten Speicher und ein Mikroprozessormodul (oder einer CPU) zur Ausführung von Applikationen. Insbesondere ist auf dem Gerät eine Verschlüsselungseinheit als Applikation installiert, die eine das Gerät identifizierende Geräte-Kennung umfasst. In dieser Ausführungsform ist das Authentisierungssystem als Client/Server-System ausgebildet. In der Regel sind eine Vielzahl von Client-Geräten und ein zentraler Server vorgesehen, wobei der Server auf mehrere Instanzen aufgeteilt sein kann und zumindest eine Registry umfasst.

Der zentrale Server ist computer-basiert und kann auch aus einem Netzwerk von Computern (z.B. Cloudsystem) bestehen oder nach einem SOA-Prinzip (Service Oriented Architecture) aufgebaut sein. Der Server ist der klinischen Einrichtung zugeordnet und steht damit im Datenaustausch. Insbesondere steht der Server in Datenaustausch mit einem Repository. Das Repository fungiert als Datenspeicher zur Speicherung von medizinischen Daten des Patienten und von anderen klinischen Daten, die dort kurz- oder langfristig gespeichert sind. Die Daten sind dort beispielsweise (jedoch nicht notwendigerweise) als Klar-Daten (unverschlüsselt) abgelegt. Von daher ist der Zugriff auf das Repository vor unberechtigten Zugriffen zu schützen.

Bei den medizinischen Daten handelt es sich um Patienten-Daten, um Untersuchungsdaten, um Berichte für einen Patienten, um Untersuchungsergebnisse, Befunde, Verordnungen oder Behandlungsanweisungen, Notfalldatensätze mit Dauerdiagnosen, Unverträglichkeiten oder Allergien, Untersuchungs- oder Behandlungstermine in unterschiedlicher Formatierung, umfassend Bilddaten, Textdaten oder Daten in anderen Formaten (z.B. Audio- und/oder Videodaten).

Die Instanziierungseinheit ist als zentrale, computer-basierte Instanz ausgebildet und ist dem Server zugeordnet. Bei der Instanziierungsinstanz kann es sich um eine Registry handeln. In einer besonderen Ausführungsform kann die Instanziierungseinheit nicht nur dem Server zugeordnet sein sondern mit diesem identisch sein, so dass die Instanziierungseinheit zusätzlich auch noch alle Funktionen des Servers übernimmt. Die Instanziierungseinheit dient zur Instanziierung der an das Authentisierungssystem anzuschließenden Geräte bzw. der zu authentisierenden Geräte. Die Instanziierungseinheit kann als Software- und/oder Hardware-Modul ausgebildet sein und steht vorzugsweise über das Mobilfunknetz in Datenaustausch mit dem zu authentisierenden elektronischen Gerät und mit einem zentralen, geschützten Speicher, der auch dem zentralen Server zugeordnet sein kann. Die Instanziierungseinheit individualisiert oder personalisiert jedes elektronische Gerät, indem ein individualisiertes Programm (Applikation) als sogenanntes "App" auf das mobile Gerät (z.B. "Handy") geladen und dort installiert wird. Nach der Installation des Apps ist das Handy eineindeutig in dem Authentisierungssystem identifizierbar und damit individualisiert. Die Instanziierung erfolgt, indem die Instanziierungseinheit einen Schlüssel und die Geräte-Kennung versteckt, also nicht lesbar, nicht vom Geräte-Anwender / Patienten erkennbar, nicht änderbar als Teil der Programme auf dem Handy installiert. Die Instanziierungseinheit verteilt somit spezifische Datensätze und ausführbare Programme auf die zu authentisierenden Geräte, um diese zu individualisieren und zu instanziieren. Desweiteren steht die Instanziierungseinheit in Datenaustausch mit einem zentralen, geschützten Speicher, der dem Server zugeordnet ist. Der Datenaustausch zwischen Instanziierungseinheit und Server kann ein digitales Netzwerk (z.B. ein Internetbasiertes Netzwerk), ein lokales Netzwerk und gegebenenfalls auch ein Mobilfunknetzwerk sein.

Bei der Applikation handelt es sich um ein Software-Modul, das als ausführbares Programm auf dem Handy installiert wird und dort unter den Programmen in einen Programmspeicher abgelegt wird. Wesentlich ist, dass die Applikation als Verschlüsselungseinheit auf dem Handy des Patienten installiert wird. Die mit der Applikation übermittelten Daten (ausführbare Datei, individueller Schlüssel, individuelle Geräte-Kennung und möglicherweise noch andere Datensätze) werden somit ausschließlich in den Programmspeicher des Handys erfasst und nicht im Datenspeicher. Der Datenspeicher des Handys ist grundsätzlich nicht gesichert und könnte leicht kompromittiert oder von unberechtigten Instanzen ausgelesen werden. Deshalb erfolgt eine Speicherung der persönlichen Daten, die von der Instanziierungseinheit an das Gerät übermittelt werden ausschließlich in versteckter Form in dem Programmspeicher des Handys.

Der Schlüssel kann Bestandteil eines symmetrischen Verschlüsselungsverfahrens sein und wird dann übereinstimmend von der Verschlüsselungseinheit auf dem Patientenhandy und von der Entschlüsselungseinheit auf dem Server verwendet. Diese (symmetrisch verschlüsselte) Ausführung erlaubt vorteilhafterweise die server-getriebene Rekonstruktion der App nach Verlust. Alternativ kann auch ein asymmetrisches Verschlüsselungsverfahren angewendet werden, wie beispielsweise basierend auf dem RSA-Algorithmus, der ein Schlüsselpaar vorsieht, bestehend aus einem öffentlichen Schlüssel (Public Key) und einem privaten Schlüssel (Private Key).

Bei der Geräte-Kennung handelt es sich um eine Geräte-spezifische oder Patienten-spezifische Kennung, die es ermöglicht, das Gerät bzw. den das Gerät bedienenden Patienten ein-eindeutig zu identifizieren. Beispielsweise kann es sich hier um die textuelle Repräsentation der Patientenidentität handeln, also um einen String mit dem Patientennamen zusammen mit eineindeutigen, invarianten Merkmalen (Geburtsdatum, Geburtsort). Alternative Ausführungen sehen hier jedoch die Zuordnung einer eineindeutigen numerischen Kennung und/oder weitere Authentisierungsdatensätze vor.

Die Signatur wird lokal auf dem Handy von der Verschlüsselungseinheit erzeugt. Beim erstmaligen Erstellen einer Signatur kann sich der Patient in dem Authentisierungssystem registrieren. Alle weiteren Anwendungen sind dann Authentisierungsvorgänge. Das Signatur-Urbild wird erzeugt, indem der Patientenname oder die Geräte-Kennung an einen Zeitstempel angehängt bzw. mit diesem verkettet wird. Anschließend wird der kombinierte Datensatz (mit der Geräte-Kennung und dem Zeitstempel) mit dem lokal gespeicherten Schlüssel verschlüsselt. Die resultierende Signatur wird dann (vorzugsweise ebenfalls ausgelöst von der Verschlüsselungseinheit) von dem lokalen Patientenhandy in Form einer Nachricht an den Server übermittelt. Die Nachricht kann Aufforderungen beinhalten zum Zugriff auf Daten und gegebenenfalls noch weitere Befehle, die Server-seitig aufgelöst werden können. Die Signatur, optional mit Nachricht, wird vom Handy über das Mobilfunknetz bzw. einem Netzwerkprovider an den Server übermittelt. Dabei wird auch die Geräte-Kennung im Klartext übertragen. Die Signatur ist hierbei die verschlüsselte Verkettung von Geräte-kennung mit dem angehängten Zeitstempel zum Zwecke der Authentisierung, die dann Server-seitig durch Entschlüsselung aufgelöst wird. Die Signatur kann noch weitere invariante Geräte - und/oder Patienten-spezifische Datensätze umfassen, wie beispielsweise demographische Datensätze des Patienten (Geburtsort, Geburtsdatum etc.) und weitere biometrische Datensätze des Patienten (wie zum Beispiel Irisdaten, Fingerabdruck etc.). Diese weiteren invarianten Datensätze werden als Kopie auch auf dem zentralen Server zugriffsgeschützt abgelegt und zur Authentisierung verwendet. Damit kann das Authentisierungsverfahren noch sicherer und robuster gegenüber Angriffen gemacht werden. Die auf dem zentralen Server bzw. direkt auf der Entschlüsselungseinheit hinterlegten Kopien der weiteren invarianten Signaturdatensätze werden dann gegenüber den entschlüsselten Signaturdatensätzen auf Übereinstimmung verglichen.

Die Entschlüsselungseinheit ist computer-basiert und kann als Software- oder Hardware-Modul ausgebildet sein. Die Entschlüsselungseinheit ist auf dem zentralen Server, insbesondere der Registry, und/oder auf der Instanziierungseinheit ausgebildet. Sie kann jedoch alternativ auch als separate Einheit an die vorstehend genannten computer-basierten Instanzen über ein Netzwerk angeschlossen sein. Die Entschlüsselungseinheit steht in Datenaustausch mit dem zentralen, geschützten Speicher, in dem eine Zuordnung zwischen Geräte-Kennung und Schlüssel abgelegt ist. Alternativ können hier noch weitere Zuordnungen abgelegt sein, um das Authentisierungsverfahren noch weiter zu verstärken. Beispielsweise kann hier zusätzlich noch eine Zuordnung zusätzlich zu den vorherstehend genannten zu einer Absenderadresse (z.B. Mobilfunknummer), einer Geräte-Identifikationsnummer, einem Namen des Geräte-Anwenders und möglicherweise weitere Identifikationsdatensätze abgelegt sein.

Der Speicher ist als zentraler, zugriffsgeschützter Speicher ausgebildet und üblicherweise dem zentralen Server und/oder der Registry zugeordnet. Zum Zugriff auf den Speicher umfasst die Entschlüsselungseinheit des Servers entweder ein Zugriffsmodul oder der Speicher ist direkt in den Server integriert. "Geschützt" meint in diesem Zusammenhang, dass ein Speicherzugriff nur von zugriffsberechtigten Instanzen und/oder Personen ausgeführt werden kann. Im einfachsten Fall ist hier ein Passwortschutz vorgesehen. Üblicherweise werden aber weitergehende Sicherungsmaßnahmen angewendet, die beispielsweise eine Verschlüsselung bzw. Entschlüsselung und eine weiterreichende Authentisierung erfordern.

Je nach Ausführungsform kann die Signatur auf unterschiedliche Weise hergestellt sein bzw. unterschiedliche Datensätze umfassen. Gemäß einer bevorzugten Ausführungsform umfasst das Signatur-Urbild einen Geräte-bezogenen Identifikationssatz, insbesondere eine Geräte-Kennung, die auf eineindeutige Weise dem jeweiligen Mobilfunkgerät des Patienten zugeordnet ist.

Gemäß einer anderen Variante der Erfindung kann das Signatur-Urbild noch Anwender-bezogene Identifikationsdatensätze umfassen, beispielsweise Datensätze, die die Identität des jeweiligen Anwenders des Gerätes (des Patienten) betreffen, wie beispielsweise die Person identifizierende Merkmale (Irisdaten etc.), wie oben bereits beschrieben. Andere Ausführungsformen sehen hier noch weitere invariante Datensätze vor, die entweder Geräte-spezifische oder Anwender-spezifisch sind. Darüber hinaus ist es möglich, eine Zufallszahl und/oder einen Zeitstempel in das Signatur-Urbild aufzunehmen. Alle im Signatur-Urbild erfassten Datensätze sind sowohl der Verschlüsselungseinheit als auch der Entschlüsselungseinheit bekannt, so dass die Einheiten die Datensätze zur Verschlüsselung und Entschlüsselung verwenden können.

Wie vorstehend bereits erwähnt, wird üblicherweise zur Verschlüsselung im Rahmen der Signaturerzeugung, eine asymmetrische Verschlüsselung der persönlichen Daten vom zentralen Server für das jeweilige Gerät vorgesehen. Dazu wird erfindungsgemäß in dem Gerät der private Schlüssel geschützt hinterlegt ("versteckt") und ein zugeordneter öffentlicher Schlüssel wird auf dem Server hinterlegt. Die Zuordnung des Schlüsselpaares (öffentlicher Schlüssel, privater Schlüssel) wird entweder von einem Drittanbieter bereitgestellt oder im zentralen Server hinterlegt.

In einer alternativen Ausprägung wird die Signatur durch symmetrische Verschlüsselung erzeugt, wobei der beiderseits zu verwendende Schlüssel ein Geheimnis darstellt und daher einerseits versteckt auf dem Gerät hinterlegt und andererseits auf der zentralen Entschlüsselungseinheit geschützt werden muss. Die den Schlüssel erzeugende Instanziierungseinheit darf diesen nicht weiter verwenden oder speichern. Diese Ausprägung hat jedoch den Vorteil, dass bei Verlust der App oder sogar des Gerätes die Entschlüsselungseinheit - nach weitergehender Authentisierung des Benutzers - diese App wieder identisch reproduzieren kann.

Gemäß einer weiteren Variante der Erfindung kann es vorgesehen sein, zwei Verschlüsselungen vorzusehen, eine erste Verschlüsselung mit einem Signatur-Verfahren (wie oben: asymmetrisch oder symmetrisch) und eine zweite Verschlüsselung mit einem symmetrischen Verfahren, um die persönlichen Daten nochmals doppelt abzusichern, die zwischen Server und Gerät ausgetauscht werden. Der erhöhte Verwaltungsaufwand wird durch den Vorteil ausgeglichen, dass eine deutlich erhöhte Sicherheit erreicht werden kann.

Wie vorstehend bereits erwähnt, ist die Instanziierungseinheit üblicherweise auf dem zentralen Server ausgebildet. Es ist auch möglich, dass die Instanziierungseinheit als Server ausgebildet ist, so dass die Instanziierungseinheit in diesem Fall mit der Funktionalität des Servers ausgebildet ist und Kommunikationsanfragen und weitere Funktionalitäten des Servers unmittelbar übernehmen kann. In diesem Fall kommunizieren die computer-basierten Einheiten (Verschlüsselungseinheit, Registry, Repository) direkt mit der Instanziierungseinheit. Diese Variante birgt den Vorteil, dass der Installationsaufwand zur Installation des Authentisierungssystems verringert werden kann, da weniger computer-basierte Einheiten notwendig sind.

Wie vorstehend bereits erwähnt, handelt es sich bei dem mobilen Gerät üblicherweise um ein Mobilfunkgerät. Das Gerät verfügt über eine programmierbare Ausführungseinheit mit entsprechenden nicht-volatilen Speicherbausteinen (EEPROM, PROM, etc.). Die Ausführungseinheit dient dazu, die Verschlüsselungseinheit als Applikation zu installieren, zu implementieren oder zu laden. Damit kann die Funktionalität der Verschlüsselungsmoduls jederzeit angepasst und erweitert werden, indem eine neue Applikation von der Instanziierungseinheit auf das Gerät geladen wird. Ein wesentlicher Vorteil ist darin zu sehen, dass jedes handelsübliche Gerät (Mobilfunkgerät) verwendet werden kann, und dass auch keinerlei Änderungen beim Mobilfunkbetreiber (z.B. den in Deutschland bekannten Mobilfunkbetreibern Vodafone, Telekom etc.) notwendig sind. Die Kommunikation zwischen Gerät und zentralem Server verläuft wie gehabt, also gegenüber etablierten Verfahren unverändert, so dass der erste spezifische Authentisierungsserver die Registry bzw. die Instanziierungsinstanz ist, die von einem Dienstleister betrieben werden kann. Im üblichen Anwendungsfall ist es vorgesehen, dass die Instanziierungseinheit als Registry fungiert und Patienten-individuelle Applikationen als Verschlüsselungseinheit auf dem Gerät installiert, in denen der Schlüssel versteckt vorliegt. Desweiteren gibt die Registry jede Applikation über die Klinik an den Patienten aus. Jeder Patient erreicht dann mit seinem Smartphone nach erfolgreicher Authentisierung über die Registry seine Patienten-spezifischen Einträge auf dem Repository. Der externe Dienstleister, der die Registry betreibt, kann unkompliziert und einfach in das Authentisierungssystem eingebunden werden, da diese Einheit nicht über schützenswertes Wissen verfügt. So hat die Registry keine Kenntnis davon, auf welchem Gerät welche Applikation geladen ist, noch kann die Registry persönliche oder zugriffsgeschützte medizinische Patienten-Daten im Klartext einsehen. Lediglich das mobile Gerät, also der Patient, und das Klinik-Repository verfügen über Klartextdaten. Alle anderen Instanzen können die Daten nicht lesen und verfügen nur über Daten in verschlüsselter Form oder nicht persönlich zuzuordnende Daten.

In einer bevorzugten Ausführungsform sind vornehmlich drei computer-basierte Instanzen vorgesehen: Zum einen das mobile Gerät, das vom Patienten bedient wird (also sein Handy, das mit der individualisierten Applikationssoftware - mit der Verschlüsselungseinheit - versehen worden ist), die Patienten-Registry, die als zentraler Server fungiert und über das Internet bzw. über einen Mobilfunkbetreiber mit den Geräten in Datenaustausch steht. Auf der Registry ist die Entschlüsselungseinheit installiert. In einer Ausführungsform kann auf der Registry auch die Instanziierungseinheit installiert sein. In einer Alternative ist die Instanziierungseinheit als separate Instanz vorgesehen. Desweitere ist als dritte Instanz das Repository als Datenhaltung vorgesehen, das insbesondere mit der Registry interagiert, um medizinische Patienten-Datensätze auszutauschen. Ein wesentlicher Vorteil dieser Architektur ist darin zu sehen, dass keine Sicherheitsmaßnahmen und Sicherungsmittel von Drittanbietern notwendig sind, um den Patienten am Kliniksystem zu authentisieren. Damit wird es für die Kliniken wesentlich leichter, Authentisierungsapplikationen zu verteilen (auf die angeschlossenen Geräte) und zu betreiben.

Gemäß einer vorteilhaften Weiterbildung der Erfindung ist die Verschlüsselungseinheit als zur Programmierung des mobilen Gerätes vorbereitete Applikation, als sogenanntes App, ausgebildet. Die Verschlüsselungseinheit überträgt die Signatur gegebenenfalls mit einer Nachricht und mit der Geräte-Kennung über einen standardisierten Kommunikationskanal (z.B. über einen Internet-basierten Hyperlink) an den Server. Umgekehrt überträgt der Server bzw. die Registry die angeforderten Daten an das Gerät, die dort auf dem Monitor ("Display") dargestellt werden können. Dieser Fall bezieht sich auf ein Download von Daten aus dem klinischen Repository. Im Falle des Daten-Downloads, also der Übertragung von medizinischen Daten vom Repository auf das mobile Gerät ist eine asymmetrische Verschlüsselung der persönlichen Daten bevorzugt, um weitere Sicherungsmaßnahmen bereitzustellen. Die übertragenen Daten werden somit nur verschlüsselt übertragen, so dass auch ein Angreifer bei einem Abfangen der Nachricht (umfassend die medizinischen Daten), diese nicht lesen kann. Darüber hinaus erhält er keine Zuordnung, zu welcher Person (Patient) diese Daten gehören. Damit kann die Sicherheit noch weiter erhöht werden.

Eine alternative Ausführungsform betrifft den Upload von Daten in das Repository. In diesem Fall werden die vorstehend genannten Schritte jeweils auf der anderen Seite (Server-Client vertauscht) ausgeführt, so dass auch das mobile Gerät medizinische Datensätze an den zentralen Speicher zur Speicherung laden kann. Beispielsweise kann diese Ausführungsform vorteilhaft sein, wenn der Patient physiologische Parameter überwachen muss (z.B. Blutdruck, Herzfrequenz, Temperatur, Blutzuckerwerte etc.). Diese Daten können dann nach erfolgreicher Authentisierung des Patienten in das Klinik-Repository geladen werden.

Um die Sicherheit noch weiter zu erhöhen, kann gemäß einem weiteren Aspekt der Erfindung eine Zeitspanne vorkonfiguriert sein, innerhalb derer der Authentisierungsvorgang abgeschlossen sein muss. Wird diese vorkonfigurierte (und jederzeit änderbare) Zeitspanne (ermittelt aus der Differenz des entschlüsselten Zeitstempels vom Gerät mit der aktuellen Server-Zeit bei Entschlüsselung) überschritten, so wird eine Fehlermeldung ausgegeben. Bedarfsweise kann der Authentisierungsvorgang nochmals neu gestartet werden. Je nach Ausführungsform kann es wie aus anderen Anwendungen (z.B. Bankanwendungen) bekannt eingestellt sein, dass nach dreimaligem Überschreiten der Zeitspanne ein Warnhinweis an den zentralen Server ausgegeben wird und keine Authentisierung mehr durchführbar ist.

Gemäß einem weiteren Aspekt der Erfindung wird automatisch erfasst, ob der Schlüssel und/oder die Geräte-Kennung unverändert, also im Original, vorliegt oder korrumpiert worden ist. Diese Ausführungsform kann mit der vorhergehenden Ausführungsform, bei der eine Sicherheitszeitspanne definierbar ist, kombiniert werden. Sofern kein Angriff bzw. keine fehlerhafte Authentisierung erkannt werden kann, ist es in einer Variante der Erfindung vorgesehen, dass (abhängig von einem erfassten Situationskontext) und/oder auf ein Bestätigungssignal hin eine Neuinstallation bzw. ein erneutes Laden der Verschlüsselungseinheit auf das Gerät veranlasst wird. In diesem Fall wird also eine neue individualisierte Verschlüsselungs-Software auf das Mobilfunkgerät des Patienten zum Zwecke der Authentisierung geladen. Somit kann gemäß einer Ausführungsvariante der Erfindung der Instanziierungsvorgang auch wiederholt ausgeführt werden.

Falls ein Angreifer den Instanziierungsvorgang unerlaubterweise manipulieren wollte, so ist es gemäß einer weiteren Variante vorgesehen, dass automatisch erfasst wird, ob die Verschlüsselungseinheit auf dem Gerät korrumpiert ist (erkennbar beispielsweise aus Abweichungen zwischen Absenderadresse und entschlüsselter Gerätekennung). In diesem Fall wird erst nach weitergehender Analyse und möglicherweise erst nach nochmaliger Authentisierung des Gerätes bzw. des Nutzers eine erneute Authentisierung ermöglicht. In der Regel ist hierzu ein weiterer Kommunikationskanal (z.B. in Schriftform oder über E-Mail bzw. über einen anderen sicheren Datenaustausch) vorgesehen, um Angriffe und Missbrauch sicher abwehren zu können.

Eine nicht beanspruchte weitere Aufgabenlösung besteht in dem elektronischen Gerät, wie vorstehend beschrieben, das zur Verwendung mit dem vorstehend beschriebenen Authentisierungssystem ausgebildet ist. Erfindungsgemäß ist auf das Gerät eine Verschlüsselungsapplikation geladen, über die sich der Anwender registrieren und authentisieren kann. Die geladene Applikation wird - wie vorstehend bereits im Rahmen der Beschreibung des Authentisierungssystems beschrieben - in den Programmspeicher in versteckter Form abgelegt und kann somit nicht vom Anwender gelesen, geändert und/oder gelöscht werden.

Das Gerät umfasst neben der Verschlüsselungseinheit vorzugsweise eine Signatureinheit, die dazu bestimmt ist, eine Signatur nach den vorstehend beschriebenen Maßnahmen zu erzeugen. Nachrichten, die von dem Gerät an den zentralen Server/Registry gesendet werden, werden ausschließlich über die Verschlüsselungseinheit mit der Signatur signiert. Der Datenaustausch ist somit in allen Fällen gesichert.

Eine weitere Aufgabenlösung besteht in einer Instanziierungseinheit, die zum Einsatz in dem Authentisierungssystem bestimmt ist. Die Instanziierungseinheit kann als Registry ausgebildet sein und ist dem zentralen Server zugeordnet. Die Instanziierungseinheit dient insbesondere dazu, die individualisierten Applikationen auf die Geräte zu verteilen und dort zu installieren. Desweiteren erfolgt seitens der Instanziierungseinheit ein Zugriff auf den zentralen, geschützten Speicher, um den Schlüssel und die Geräte-Kennung und/oder deren Zuordnung so abzulegen, dass die Entschlüsselungseinheit Nachrichten vom Gerät entschlüsseln kann und aufgrund eines Vergleichs der Geräte-Kennungen authentisieren kann. Die Instanziierungseinheit kann als separate Einheit ausgebildet sein und verfügt über Schnittstellen zum Datenaustausch, insbesondere über eine Mobilfunkschnittstelle und weitere Netzwerkschnittstellen. Sie ist in der Regel computer-gestützt. Eine weitere Aufgabenlösung besteht in einem Authentisierungsverfahren gemäß dem beiliegenden Patentanspruch. Das Verfahren gliedert sich in eine Instanziierungsphase und in eine Authentisierungsphase.

In der Instanziierungsphase werden die Geräte instanziiert, über die sich Patienten registrieren und am zentralen KlinikSystem authentisieren können. In der Instanziierungsphase werden die Geräte mit der Verschlüsselungssoftware individualisiert instanziiert. Dazu wird die Verschlüsselungssoftware auf dem Gerät installiert bzw. geladen. Der Benutzer des Gerätes kann sich bei erstmaliger Benutzung am Gerät registrieren und anschließend Authentisierungsvorgänge ausführen.

In der Authentisierungsphase erfolgt die eigentliche Authentisierung für einen Datenzugriff auf Daten des Kliniksystems (umfassend Upload und Download von medizinischen Daten). Ebenso ist jeglicher Nachrichtenaustausch zwischen Gerät und Klinik-Repository (über die Registry) umfasst.

Auf dem Gerät werden folgende Verfahrensschritte zur Authentisierung ausgeführt:
- Zunächst wird eine Signatur erzeugt. Die Signatur umfasst mindestens eine verschlüsselte Form einer Verkettung von der Geräte-Kennung und einem Zeitstempel.
- Die Signatur wird mit der Geräte-Kennung und gegebenenfalls mit einer Nachricht von dem Gerät an die Registry versendet.
- Die Signatur wird zusammen mit der Gerätekennung und gegebenenfalls einer Nachricht versendet und optional symmetrisch verschlüsselt und stattdessen wird das Verschlüsselungsergebnis gesendet.

Seitens der Registry werden folgende Schritte zur Authentisierung ausgeführt:
- Die Nachricht mit der Signatur und der Geräte-Kennung werden empfangen und bedarfsweise (optional) symmetrisch entschlüsselt.
- Die Geräte-Kennung wird erfasst.
- Mit der erfassten Geräte-Kennung erfolgt ein Zugriff auf den zentralen, geschützten Speicher, um den jeweils korrespondierenden (der Geräte-Kennung zugeordneten) Schlüssel auszulesen.
- Mittels des ausgelesenen Schlüssels erfolgt eine Entschlüsselung der Signatur.
- Als Entschlüsselungsergebnis wird die entschlüsselte Geräte-Kennung mit Zeitstempel ausgelesen. Nun kann die Entschlüsselungseinheit das Entschlüsselungsergebnis mit der empfangenen Geräte-Kennung auf Übereinstimmung vergleichen. Bei Übereinstimmung gilt der Authentisierungsvorgang als erfolgreich und es kann ein Zugriff auf das Repository ausgeführt werden. Um die Datensätze in dem Repository zu finden, werden die Geräte-Kennung und/oder weitere, der Geräte-Kennung zugeordnete identifizierende Kennsätze verwendet.

Die Signatur wird somit als Teil jeder Nachricht in der Art von Metadaten oder als Envelope mit gesendet.

Gemäß einem Aspekt der Erfindung umfasst das Verfahren einen Vergleich des Entschlüsselungsergebnisses mit der Geräte-Kennung auf Übereinstimmung. Der Vergleich umfasst vorzugsweise einen Vergleich auf ein zeichenweises Enthaltensein der Geräte-Kennung im Signatur-Urbild.

Gemäß einer bevorzugten Ausführungsform umfasst der Authentisierungsvorgang noch einen zusätzlichen Vergleich, nämlich den Vergleich der Uhrzeiten. Falls die Geräte-Kennung übereinstimmt, so dass der Authentisierungsvorgang als erfolgreich gilt, aber die Zeiten, also der Zeitstempel, nicht übereinstimmen, so sollte das Gerät über ein Warnsignal informiert werden, dass die Uhrzeiten aktualisiert werden müssen. Gemäß einem Aspekt der Erfindung ist es in diesem Fall jedoch auch vorgesehen, dass die Abweichung der Zeitstempel möglicherweise auf eine Kompromittierung der Nachricht deuten könnte. Deshalb wird auch der Server mit einer Meldung informiert, dass hier möglicherweise eine Kompromittierung erfolgt ist. Bedarfsweise können hier weitere Analyseschritte getriggert werden.

Im Hinblick auf die Analyse der Zeitstempel kann eine vorkonfigurierbare Abweichung zwischen den Zeitstempeln konfiguriert werden. Beispielsweise kann voreingestellt sein, dass eine Abweichung von 10 Minuten noch tolerierbar ist, während darüber hinaus gehende Abweichungen die Ausgabe eines Warnsignals auslösen.

Falls der Authentisierungsvorgang erfolgreich abgeschlossen werden konnte, können weitere sichere Kanäle geöffnet werden, um einen Datenaustausch bzw. eine direkte Kommunikation zwischen Gerät und Repository zu ermöglichen. Beispielsweise kann hierzu ein Schlüssel mit begrenzter Gültigkeit bereitgestellt werden. Mittels dieses Schlüssels können dann autorisierungsbedürftige, zu schützende Daten über den Kanal zwischen Gerät und Repository ausgetauscht werden, solange die Gültigkeit des Schlüssels vorliegt. Mit anderen Worten können nach erfolgreicher Authentisierung auch andere sichere Kommunikationskanäle, gegebenenfalls auch zu anderen Speichern und/oder Instanzen geöffnet werden.

Üblicherweise ist es vorgesehen, dass der Authentisierungsvorgang vom Client (Patient), also Geräte-seitig initiiert wird. Alternative Ausführungsformen sehen hier jedoch auch eine Initiierung des Authentisierungsvorganges seitens der Registry oder des Repositories vor. Dies ist beispielsweise dann denkbar, falls der Patient über das Versenden einer diesbezüglichen Nachricht von der Registry und/oder dem Repository an das Gerät daran erinnert werden soll, Daten abzufragen und/oder hochzuladen.

Der Datenaustausch zwischen Gerät und Repository erfolgt ausschließlich verschlüsselt. Dabei werden entweder feste, gerätespezifische oder sogar gerätespezifische, temporär gültige Schlüssel verwendet. Auch bei einem Abfangen der Nachricht kann somit ein Angreifer die übertragenen Daten nicht im Klartext einlesen.

Üblicherweise ist es vorgesehen, dass ein Patient über ein Mobilfunkgerät verfügt, das er allein bedient. In diesem Fall ist eine 1:1-Zuordnungsrelation zwischen User (Patient) und Gerät vorgesehen. Alternative Ausführungsformen sehen jedoch vor, dass eine Gruppe von Patienten über ein Gerät ansprechbar ist. In diesem Fall ist eine Zuordnungsrelation von n:1 zwischen Patient und Gerät vorgesehen. Alternativ ist es auch möglich, dass ein Patient über mehrere Geräte verfügt, die er zur Authentisierung verwenden kann. In diesem Fall wird die Verschlüsselungseinheit als Applikation auf alle Authentisierungs-Geräte implementiert. In diesem Fall ist eine 1:n-Zuordnungsrelation zwischen Patient und Gerät eingestellt.

Eine weitere Aufgabenlösung besteht in einem Computerprogrammprodukt. Das Produkt umfasst Computerprogrammcode, der zur Durchführung aller Verfahrensschritte des vorstehend beschriebenen oder beanspruchten Verfahrens bestimmt ist, wenn das Computerprogramm bzw. der Computerprogrammcode auf dem Computer ausgeführt wird. Dabei kann das Computerprogramm auch auf einem Maschinen- oder Computerlesbaren Speichermedium gespeichert sein. Eine Alternative sieht vor, dass das Computerprogramm über ein Netzwerk als ausführbare Einheit eingelesen wird.

Ebenso ist es möglich, dass das Verfahren auf unterschiedliche Computerinstanzen, insbesondere auf eine Verschlüsselungseinheit und eine Entschlüsselungseinheit sowie auf eine Instanziierungseinheit, aufgeteilt wird. Einzelne Schritte des Verfahrens können dann auf den einzelnen Einheiten (Ver- und Entschlüsselungseinheit) ausgeführt werden, so dass das Verfahren und das Authentisierungssystem als verteiltes System implementiert ist. Die bevorzugte Ausführungsform bezieht sich auf eine Software-Implementierung. Alternative Ausbildungen sehen hier eine teilweise Hardware-gestützte Implementierung vor.

Die vorstehend beschriebenen, erfindungsgemäßen Ausführungsformen des Verfahrens können auch als Computerprogrammprodukt mit einem Computerprogramm ausgebildet sein, wobei der Computer zur Durchführung des oben beschriebenen, erfindungsgemäßen Verfahrens veranlasst wird, wenn das Computerprogramm auf dem Computer bzw. auf einem Prozessor des Computers ausgeführt wird.

Eine alternative Aufgabenlösung besteht auch in einem Computerprogramm mit Computer-Programmcode zur Durchführung aller Verfahrensschritte des beanspruchten oder oben beschriebenen Verfahrens, wenn das Computerprogramm auf dem Computer ausgeführt wird. Dabei kann das Computerprogramm auch auf einem maschinenlesbaren Speichermedium gespeichert sein.

Eine alternative Aufgabenlösung sieht ein Speichermedium vor, das zur Speicherung des vorstehend beschriebenen, computerimplementierten Verfahrens bestimmt ist und von einem Computer lesbar ist.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:
- Figur 1: eine Prinzipdarstellung des erfindungsgemäßen Authentisierungssystem mit ausgewählten Instanzen,
- Figur 2: eine schematische Darstellung einer Instanziierungseinheit und deren Kommunikationskontext im Rahmen eines erfindungsgemäßen Authentisierungsvorganges,
- Figur 3: eine schematische Darstellung von Daten und/oder Nachrichten, die beim Datenaustausch mit einem zentralen Server übertragen werden und
- Figur 4: eine schematische Darstellung eines Ablaufs gemäß einer bevorzugten Ausführungsform des erfindungsgemäßen Authentisierungsverfahrens.

Im Folgenden wird die Erfindung anhand der Figuren näher erläutert. In den Figuren sind unterschiedliche Ausführungsformen der Erfindung dargestellt, dabei sind die Bezugszeichen für dieselben Module bzw. Bauteile auch in den jeweils anderen Figuren übereinstimmend verwendet.

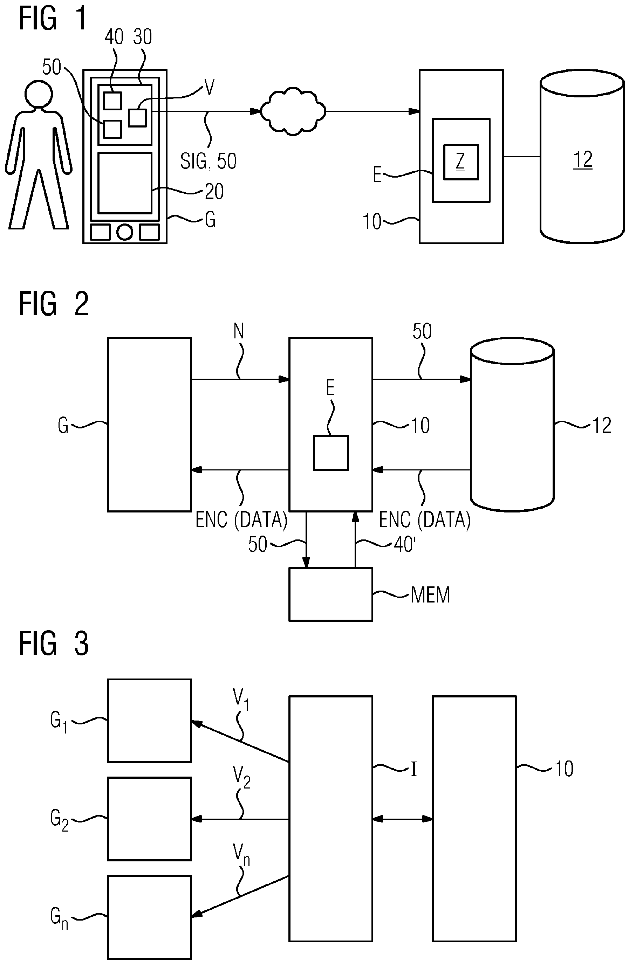

Im Folgenden wird der Kontext eines Authentisierungsvorganges unter Bezugnahme auf Figur 1 näher erläutert: Die Erfindung betrifft ein Authentisierungssystem zur Authentisierung von vielen mobilen, elektronischen Geräten G, die von Patienten oder medizinischem Personal betrieben werden. In der bevorzugten Ausführungsform handelt es sich bei den Geräten um Handys oder andere Mobilfunkgeräte G. Die Mobilfunkgeräte oder Smartphones G stehen über das Internet und/oder über ein Mobilfunknetz (das von einem beliebigen Mobilfunknetzbetreiber betrieben wird) in Datenaustausch mit einem zentralen Server. Der Server ist in der bevorzugten Ausführungsform als Registry 10 ausgebildet. Die Registry 10 ist üblicherweise einer Klinik oder einem Krankenhausverband, einer Arztpraxis oder einem Verbund von Arztpraxen zugeordnet. Sie kann von einem externen Dienstanbieter betrieben werden. Die Registry 10 steht wiederum in Datenaustausch mit einem Repository 12. Auf dem Repository 12 sind medizinische Datensätze abgelegt. Beispielsweise können hier Untersuchungsergebnisse, Befunde, Bilddaten von radiologischen Untersuchungen, wissenschaftliche Studien, Verordnungen, Termine etc. abgelegt sein. Das Repository 12 ist als zentraler, und vorzugsweise auch als zugriffsgeschützter Speicher ausgebildet. In einer alternativen Ausführungsform kann das Repository 12 auch einen separaten Speicherbereich umfassen, der als geschützter Speicher ausgebildet ist. In einer bevorzugten Ausführungsform umfasst die Registry 10 einen zentralen, geschützten Speicher mit authentisierungsbedürftigen und unter Zugriffsschutz stehenden persönlichen, vertraulichen Patienten-Daten. Wie in Figur 1 durch den Pfeil dargestellt, stehen die Registry 10 und das Repository 12 in Datenaustausch miteinander. Hier sind unterschiedliche Kommunikationskanäle denkbar. Bevorzugt wird ein Datenaustausch über ein Netzwerk (WLAN oder LAN). Alternativ kann hier auch eine Internetverbindung über eine bestimmbare Version aus der Internetprotokollfamilie (z.B. http als Anwendung des TCP/IP-Protokolls) installiert sein. Die erfindungsgemäße Architektur des Authentisierungssystems ist Mobilfunkgeräte-basiert. Mit anderen Worten wird davon ausgegangen, dass ein Patient oder eine Gruppe von Patienten über ein Mobilfunkgerät G verfügt. Das Mobilfunkgerät G ist allerdings erfindungsgemäß in seinen Gerätekomponenten modifiziert, indem es mit einer individualisierten Softwareapplikation versehen wird, die auf das Gerät aufgespielt wird. Die Gerätekomponenten sind deshalb erfindungsgemäß auch abweichend adressiert. So wird das Versenden einer Nachricht N an die Registry 10 beispielsweise ausschließlich über die installierte Verschlüsselungseinheit V ausgeführt.

Der vorstehend erwähnte Instanziierungsvorgang wird im Folgenden unter Bezugnahme auf Figur 2 näher erläutert. Erfindungsgemäß ist eine Instanziierungseinheit I vorgesehen, die in Datenaustausch mit den zu authentisierenden Geräten G steht. Die Datenverbindung zwischen Gerät G und Instanziierungseinheit I ist vorzugsweise Mobilfunk-basiert. Alternativ können hier jedoch auch andere Datenübertragungsnetzwerke herangezogen werden. Bei den Geräten G handelt es sich um handelsübliche Mobilfunkgeräte unterschiedlicher Art und Bauweise. Die Instanziierungseinheit ist eine computer-basierte Einheit, die zur Instanziierung der Geräte G bestimmt ist und dazu eine personalisierte und/oder Geräte-spezifische Applikation als Verschlüsselungseinheit V auf das zu authentisierende Gerät G installiert bzw. lädt. Dies ist in Figur 2 dadurch gekennzeichnet, dass die Pfeile (die eine Datenübertragung repräsentieren sollen), die von der Instanziierungseinheit I ausgehen und auf die Geräte G₁, G₂.... Gₙ zeigen unterschiedliche Versionen von Verschlüsselungseinheiten V, nämlich eine erste Verschlüsselungseinheit V₁, eine zweite Verschlüsselungseinheit V₂ etc. repräsentieren. In der bevorzugten Ausführungsform ist eine 1:1-Zuordnung zwischen Verschlüsselungseinheit V und Gerät G vorgesehen, so dass eine erste Verschlüsselungseinheit V₁ auf dem ersten Gerät G₁ installiert wird, eine zweite Verschlüsselungseinheit V₂ auf dem zweiten Gerät G₂... und eine n-te Verschlüsselungseinheit Vₙ auf dem n-ten Gerät Gₙ. Alternativ können jedoch hier auch andere Zuordnungen gewählt werden, so dass beispielsweise ein und dieselbe Verschlüsselungseinheit V auf mehrere Geräte G verteilt und dort installiert wird (dies entspricht einer 1:N-Zuordnung zwischen Verschlüsselungseinheit und Gerät). Alternativ ist es auch möglich, hier andere Anwendungsszenarien abzudecken, so dass mehrere Verschlüsselungseinheiten V auf einem Gerät G installiert werden (dies entspricht einer M:1-Zuordnung zwischen Verschlüsselungseinheit und Gerät). Üblicherweise ist davon auszugehen, dass sich ein Anwender (üblicherweise ein Patient) an seinem Mobilfunkgerät durch die Eingabe von Identifikations- und/oder Authentisierungssignalen registriert. Üblicherweise sind hier eine Passworteingabe, ein Fingerprint oder sonstige Registrierungsmaßnahmen vorgesehen. Damit kann der zentrale Server, die Registry 10 und/oder die Instanziierungseinheit I über das jeweilige Gerät G auch den spezifischen Patienten P erreichen. Im Folgenden ist von einer individualisierten Geräte-spezifischen Applikation die Rede, die als Verschlüsselungseinheit V auf dem Gerät G installiert wird. Da aber üblicherweise ein spezifischer Patient ein bestimmtes Gerät betreibt, bezieht sich die Erfindung auch darauf, dass die Applikation Userspezifisch ist, die als Verschlüsselungseinheit V auf dem Gerät G installiert wird. Die Instanziierung, die von der zentralen Instanziierungseinheit I ausgelöst wird, überträgt als Verschlüsselungseinheit V eine Softwareapplikation an das jeweilige Gerät G. Die Applikation umfasst einen Schlüssel 40 und eine eineindeutige Geräte-Kennung 50. Der Schlüssel ist Teil eines kryptologischen Verfahrens und kann als symmetrischer Schlüssel oder als asymmetrisches Schlüsselpaar 40, 40' ausgebildet sein. Sowohl der Schlüssel 40 als auch die Geräte-Kennung 50 werden in versteckter Form in einem Programmspeicher 30 des Gerätes G abgelegt. Dies ist ein wesentliches Merkmal der Erfindung, da zu speichernde Daten nicht in einem Datenspeicher 20 abgelegt werden, wie dies üblicherweise der Fall ist (etwa für Audiodaten, Bilddaten etc.), sondern in einem zugriffsgeschützten Zugriffsmodul des Gerätes G, nämlich in dem Programmspeicher 30. Die Geräte-Kennung 50 ist für das jeweilige Gerät G identifizierend. Mit anderen Worten gibt es eine bijektive Abbildung zwischen Geräte-Kennung 50 und Gerät G und das Gerät G kann über die Geräte-Kennung 50 eineindeutig identifiziert und adressiert werden. Aufgrund der Tatsache, dass die Authentisierungsdatensätze (insbesondere Schlüssel 40 und Geräte-Kennung 50) in versteckter Form auf dem Gerät G abgelegt werden, kann auch der Patient bzw. der Geräte-User keinen Zugriff auf die Geräte-Kennung oder den Schlüssel 40 erhalten. Darüber hinaus kann er diese Datensätze nicht modifizieren und auch nicht löschen.

Nachdem die Verschlüsselungseinheit V lokal auf dem Gerät G installiert worden ist, kann sich der Anwender beim ersten Mal über die Verschlüsselungseinheit V einem Registrierungsvorgang unterziehen. Anschließend kann die Verschlüsselungseinheit V verwendet werden, um Nachrichten zu signieren und damit den Patienten an der zentralen Registry 10 zu authentisieren, um die Zugriffe auf das Repository 12 in authentisierter Form durchführen zu können. Dazu erzeugt die Verschlüsselungseinheit V vor jedem Nachrichtenversand ein Signatur-Urbild SIG-UB. Dieses Signatur-Urbild wird von der Verschlüsselungseinheit V erzeugt, indem die Geräte-Kennung 50 und ein Zeitstempel 60 konkateniert werden. Der konkatenierte Datensatz wird dann mit dem Schlüssel 40 verschlüsselt. Alternativ kann das Signatur-Urbild SIG-UB auch noch weitere Authentisierungsdatensätze 51 umfassen. Die weiteren Authentisierungsdatensätze 51 können beispielsweise invariante Patienten-spezifische Datensätze (demographische Daten, biometrische Daten etc.) sein. In diesem Fall werden die Geräte-Kennung 50, der Zeitstempel 60 und die weiteren Authentisierungsdatensätze 51 miteinander verknüpft und anschließend verschlüsselt. Bei allen Nachrichten N, die von dem Gerät G an den zentralen Server bzw. an die Registry 10 zu übertragen sind, werden mit der Signatur SIG - also der Verschlüsselung von SIG-UB - signiert. Im Rahmen eines Kommunikationsvorganges zwischen Gerät G und Registry 10 empfängt die Registry 10 dann die Nachricht N mit der Signatur SIG und der Geräte-Kennung 50. Dies ist in Figur 1 mit dem Datenpaket dargestellt, das vom Gerät G an die Registry 10 gesendet wird, das mit dem Oval "{SIG}, 50" repräsentiert ist. Der Datenaustausch erfolgt auch hier bevorzugt über das Internet.

Um dieses Datenpaket bzw. die Nachricht N serverseitig empfangen zu können, ist die Registry 10 erfindungsgemäß um eine Entschlüsselungseinheit E erweitert. Die Entschlüsselungseinheit E ist auf der Registry 10 installiert und umfasst ein Zugriffsmodul Z, mit dem ein Zugriff auf einen zentralen, geschützten Speicher MEM ausführbar ist. Alternativ umfasst die Entschlüsselungseinheit E auch direkt und unmittelbar den sicheren Speicher MEM. Die Entschlüsselungseinheit E ist vorzugsweise als Software-Modul ausgebildet und dient zum Empfangen der, vom Gerät G gesendeten Nachricht bzw. gesendeten Signatur SIG mit der Geräte-Kennung 50. Desweiteren dient die Entschlüsselungseinheit E dazu, den eigentlichen Authentisierungsvorgang auszuführen. Dies erfolgt über den Vergleich eines Signatur-Urbildes als Entschlüsselungsergebnis SIG-UB mit der übertragenen Geräte-Kennung 50. Bei Übereinstimmung des Entschlüsselungsergebnisses mit der Geräte-Kennung 50 gilt das Gerät G bzw. der jeweilige Zugriff als authentisiert. Anderenfalls kann eine Fehlermeldung ausgegeben werden und der Zugriff wird nicht ausgeführt. Die Registry 10 löst die übertragene Nachricht auf und erhält nach Entschlüsselung das Signatur-Urbild SIG-UB, umfassend den Zeitstempel 60 in Klartext, sowie die Geräte-Kennung 50 ebenfalls in Klartext. Die entschlüsselten Datensätze (Zeitstempel und Geräte-Kennung) werden als Entschlüsselungsergebnis vorübergehend zwischengespeichert. Sobald die Geräte-Kennung 50 aus dem Entschlüsselungsergebnis mit der übertragenen Geräte-Kennung übereinstimmt, gilt der Authentisierungsvorgang als erfolgreich ausgeführt.

Alternative Ausführungen sehen hier noch weitere Kontrollmaßnahmen vor. Beispielsweise ist es möglich, die Zeitangaben aus dem Zeitstempel 60 zu analysieren, um zu überprüfen, ob die Zeitmarke des Gerätes G nur um einen vorkonfigurierbaren Differenzbereich von der Zeitmarke der Registry 10 abweicht. Mit anderen Worten sollte die Registry-Zeit und die Geräte-Zeit nur um ein vorkonfigurierbares Toleranzmaß voneinander abweichen. Nach erfolgreicher Authentisierung kann dann die Registry 10 einen Zugriff auf das Repository 12 ausführen. Der Zugriff wird in der Regel über die Geräte-Kennung 50 indiziert, um die Patienten-spezifischen und relevanten Datensätze in dem Repository 12 zu finden und an das Gerät G des Patienten zu übermitteln.

Im Folgenden werden die ihm Rahmen der Authentisierung übertragenen Datensätze unter Bezugnahme auf Figur 3 näher erläutert. Nach Instanziierung des Gerätes G überträgt dieses die Nachricht N, umfassend die Signatur SIG und die Geräte-Kennung 50 an die Entschlüsselungseinheit E der Registry 10 (oberster Pfeil auf der linken Seite). Daraufhin wird die Entschlüsselungseinheit E Server-seitig aktiv und entschlüsselt das Ergebnis und führt den Authentisierungsvorgang durch. Dazu greift die Entschlüsselungseinheit E mit der Geräte-Kennung 50 auf den sicheren Speicher MEM zu, um einen Entschlüsselungsschlüssel 40' auszulesen. In einer alternativen Ausbildung ist der Schlüssel 40' nicht auf dem Repository 12 abgelegt (dies ist auch möglich, wenn der Speicherbereich zugriffsgeschützt ist), sondern auf einem separaten Speicher, auf den die Registry 10 Zugriff hat. Der Entschlüsselungsschlüssel 40' ist auf eineindeutige Weise dem Verschlüsselungsschlüssel 40 zugeordnet und Teil einer asymmetrischen Verschlüsselung. In der Regel handelt es sich um ein asymmetrisches Schlüsselpaar, umfassend einen privaten und einen öffentlichen Schlüssel. Dabei ist der private Schlüssel uneinsehbar und versteckt im Programmspeicher 30 des Gerätes G abgelegt, während der öffentliche Schlüssel 40' in einem zentralen Speicher abgelegt ist. In dieser Ausführungsform umfasst der Speicher (beispielsweise das Repository 12) eine Zuordnungstabelle zwischen Geräte-Kennung 50 und Schlüssel 40, 40'. Nach Auslösen des jeweils zugeordneten Entschlüsselungsschlüssels 40' wird dieser an die Entschlüsselungseinheit E weitergeleitet. Daraufhin kann die Entschlüsselungseinheit E die Signatur SIG entschlüsseln, resultierend in dem Signatur-Urbild SIG-UB, um daraufhin den Authentisierungsvorgang durch Vergleich (wie vorstehend bereits beschrieben) durchzuführen. Bei erfolgreicher Authentisierung kann nochmals ein Zugriff auf das Repository 12 ausgeführt werden, um den konkret angeforderten Datensatz einzulesen und vorzugsweise in verschlüsselter Form (üblicherweise mit dem öffentlichen Schlüssel 40') zu verschlüsseln und über die Registry 10 an das Gerät G zu übertragen. Die verschlüsselten Daten sind in Figur 3 mit der ovalen Kennzeichnung "ENC (DATA)" gekennzeichnet.

Der vorstehend beschriebene Vorgang bezieht sich auf ein Daten-Download von medizinischen Datensätzen auf das Gerät G. Das erfindungsgemäße Authentisierungsverfahren ist jedoch ebenso auf einen Daten-Upload zu verwenden, bei dem der Patient über sein Gerät G Datensätze an das zentrale Repository 12 zur Speicherung sendet. Beispielsweise kann es sich hier um aktuelle Messwerte des Patienten für Blutdruck, Blutglucosespiegel, Temperatur etc. handeln.

In einer weiteren vorteilhaften und auch bevorzugten Ausführungsform wird anstelle der Geräte-Kennung 50 eine Patienten-Nummer in versteckter Form in den Programmspeicher 30 auf dem Gerät G gespeichert und als Teil der Nachricht N an die Registry 10 zur Authentisierung übertragen. Bei dem ebenfalls im Programmspeicher 30 abgelegten Schlüssel handelt es sich um einen geheimen Schlüssel des Patienten (Private Key). Als Teil jeder Kommunikation bzw. jeder Datenanfrage sendet die Verschlüsselungseinheit V dann die signierte Konkatenation aus Patienten-Nummer und Zeitstempel 60 (wobei sowohl die Patienten-Nummer also auch der Zeitstempel 60 z.B. mit einem symmetrischen Verschlüsselungsverfahren nachverschlüsselt ist) an die Registry 10. Der Zeitstempel 60 wird deshalb verschlüsselt als Teil der Signatur SIG bei jeder Nachricht übertragen, um einen veränderlichen Anteil der Nachricht zu haben, die es seitens des Servers bzw. der Entschlüsselungseinheit E aufzulösen gilt, um Angriffe von unberechtigten Personen - die eine Kopie einer früheren SIG verwenden könnten - besser abwehren zu können.

Als wesentlicher Vorteil der Erfindung lässt sich festhalten, dass keinerlei Änderungen am Gerät G notwendig sind. Mit anderen Worten ist es lediglich notwendig, die Verschlüsselungsapplikation V auf dem Gerät G zu installieren. Es gibt keine weiteren Anforderungen an das Gerät G, so dass hier auch handelsübliche Mobilfunkgeräte verwendet werden können. Desweiteren sind auch keine spezifischen Vorkehrungen beim Mobilfunkbetreiber notwendig.

Ein weiterer Vorteil, der die Sicherheit des erfindungsgemäßen Authentisierungssystems erhöhen kann ist darin zu sehen, dass keine unverschlüsselten medizinischen Datensätze zwischen Gerät G und Repository 12 übertragen werden. Lediglich der Patient als Client des Gerätes G und das Klinik-Repository 12 verfügen über Datensätze im Klartext. Die ausgetauschten Datensätze werden ausschließlich verschlüsselt übertragen.

Vorzugsweise ist es vorgesehen, dass der öffentliche Schlüssel ebenfalls in einem zentralen geschützten Speicher MEM abgelegt ist, der der Entschlüsselungseinheit E und damit der Registry 10 zugeordnet ist. Üblicherweise wird der Schlüssel in der Registry 10 vorgehalten. Um die Daten, die vom Gerät G angefordert wurden und nach erfolgreichem Authentisierungsvorgang von dem Repository 12 an das Gerät G zu senden sind, mit dem öffentlichen Schlüssel des Gerätes G bzw. des anfragenden Patienten P verschlüsselt. Dazu greift das Repository 12 auf den öffentlichen Schlüssel der Registry 10 zu. Sobald die Daten dann auf dem Gerät empfangen werden, kann die Verschlüsselungseinheit V dazu ausgebildet sein, die verschlüsselten Daten mit dem geheimen oder privaten Schlüssel zu entschlüsseln.

Eine alternative Ausführungsform sieht die Verwendung eines weiteren Schlüsselpaares vor, um die Sicherheit noch weiter zu erhöhen. Hierbei kann es sich nochmals um ein asymmetrisches oder um ein symmetrisches Verfahren handeln, um die persönlichen Daten zu verschlüsseln.

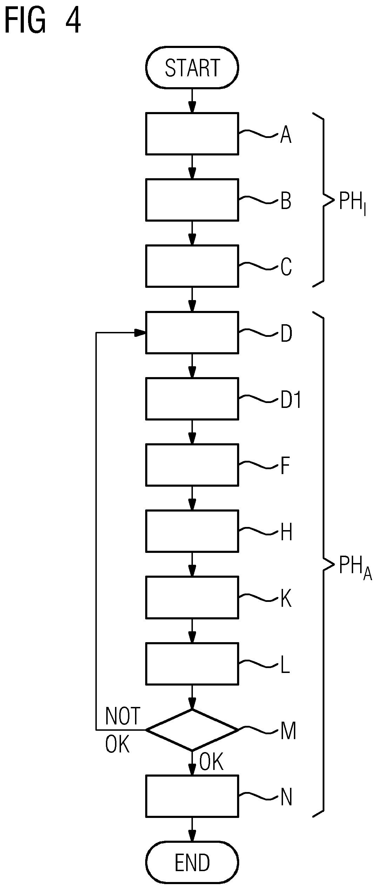

Im Folgenden wird ein Ablauf eines Authentisierungsvorganges gemäß einer bevorzugten Ausführungsform unter Bezugnahme auf Figur 4 näher erläutert.

Nach dem Start des Authentisierungsvorganges gliedert sich das Verfahren in eine Instanziierungsphase und in eine Authentisierungsphase. Zur Durchführung der Authentisierungsphase ist der erfolgreiche Abschluss der Instanziierung erforderlich.

Die Instanziierung bezieht sich auf die Instanziierung der Geräte G. Dies ist in Figur 4 mit dem Verfahrensschritt A gekennzeichnet. Zur Instanziierung wird auf dem Gerät G eine Verschlüsselungseinheit V als Softwareapplikation installiert, die eine Geräte- oder Patienten-Kennung 50 und einen Schlüssel 40 in versteckter Form auf dem Programmspeicher 30 des Gerätes speichert. Die Applikation dient zur Ver- und Entschlüsselung von medizinischen Datensätzen, die mit dem Repository 12 ausgetauscht werden. Der Installationsvorgang ist in Figur 4 mit dem Bezugszeichen B gekennzeichnet.

Anschließend kann sich der Client oder Patient bzw. User an seinem Gerät G registrieren. Dies ist mit dem Verfahrensschritt C gekennzeichnet.

Zunächst müssen alle Geräte G instanziiert werden, um später für einen Authentisierungsvorgang zur Verfügung zu stehen.

In der Authentisierungsphase kann sich ein Patient über sein Mobilfunkgerät G an der zentralen Registry 10 authentisieren und identifizieren.

Dazu wird in Verfahrensschritt D eine Signatur erzeugt. Das Signatur-Urbild SIG-UB setzt sich zusammen aus einer Geräte-oder Patienten-Kennung 50 und einem Zeitstempel 60. Beide Datensätze bilden das Signatur-Urbild SIG-UB, dieses wird mit einem Schlüssel 40 verschlüsselt und bildet so die Signatur SIG.

In Schritt D1 wird eine Nachricht N mit Signatur SIG und Geräte-Kennung 50 an die Registry 10 gesendet.

In Verfahrensschritt F wird die Nachricht N Server-seitig auf der Registry 10 empfangen.

In Schritt H wird die übertragene Geräte-Kennung 50 erfasst. Im Folgenden wird die Ausführungsform beschrieben, in der die Geräte-Kennung 50 übertragen wird. Wie vorstehend bereits erwähnt, ist es jedoch auch möglich, anstelle der Geräte-Kennung 50 eine Patienten-Identifikationsnummer zu übertragen, die auf eineindeutige Weise den jeweiligen Patienten identifiziert.

Im Verfahrensschritt K erfolgt ein Zugriff auf den zentralen geschützten Speicher MEM, der der Registry 10 zugeordnet ist. Der Zugriff dient dazu, den Entschlüsselungsschlüssel 40' auszulesen, der der Geräte-Kennung 50 zugeordnet ist. Der Entschlüsselungsschlüssel 40 dient zur Entschlüsselung der empfangenen Nachricht N. Im anschließenden Verfahrensschritt L wird die Nachricht N mit der empfangenen Signatur SIG entschlüsselt unter Verwendung des Schlüssels 40'. Dabei wird ein Signatur-Urbild SIG-UB als Entschlüsselungsergebnis erzeugt und mit der ausgelesenen Geräte-Kennung 50 auf Übereinstimmung verglichen.

Der Vergleich des Entschlüsselungsergebnisses mit der Geräte-Kennung 50 erfolgt im Verfahrensschritt M.

Bei Übereinstimmung erfolgt in Schritt N ein Zugriff mit der ausgelesenen Geräte-Kennung 50 auf das Repository 12 zum Auslesen der jeweiligen medizinischen Daten. Daraufhin kann das Verfahren enden.

Wie in Figur 4 dargestellt erfolgt der Zugriff auf das Repository 12 nur, falls die Authentisierung erfolgreich war. Dies ist mit dem Oval gekennzeichnet, das die Bezeichnung "OK" trägt. Andernfalls kann das Verfahren entweder unmittelbar enden oder der Authentisierungsvorgang wird wiederholt, was in Figur 4 mit dem nach oben weisenden Pfeil repräsentiert ist, so dass das Verfahren bei Verfahrensschritt D nochmals beginnt.

Grundsätzlich sind zwei unterschiedliche Ausführungsformen möglich, die ein unterschiedliches Verschlüsselungssystem vorsehen: Ein symmetrisches oder ein asymmetrisches Verfahren. Alternativ können auch Kombination angewendet werden. Bei der symmetrischen Verschlüsselung stimmt der Schlüssel 40 identisch mit dem Schlüssel 40' überein. Bei der asymmetrischen Verschlüsselung ist der Schlüssel 40 vorzugsweise ein privater Schlüssel und der Schlüssel 40' der zugehörige öffentliche Schlüssel des Schlüsselpaares.

Zusammenfassend kann die Erfindung mit einer Patienten-Authentisierung beschrieben werden, deren Architektur auf einem herkömmlichen Mobilfunkgerät (Handy) basiert, das mit einer zentralen Registry 10 und einer Instanziierungseinheit I in Datenaustausch steht. Die Instanziierungseinheit I installiert individualisierte Apps auf dem Handy des Patienten. Die Authentisierungsdatensätze, wie Schlüssel 40, 40' und Geräte-Kennung 50 sind sowohl lokal auf dem Handy als auch in einem zentralen geschützten Speicher MEM der Registry 10 vorgehalten. Sowohl auf dem Handy als auch in der zentralen Registry 10 sind Software-Applikationen installiert (Verschlüsselungseinheit V und Entschlüsselungseinheit E), die den Authentisierungsvorgang nach Übertragen einer Nachricht N von Gerät G an Registry 10 ausführen. Die medizinischen Datensätze liegen auf dem Klink-Repository 12 und sind nach erfolgreicher Authentisierung über die Registry 10 auch für das mobile Gerät G verfügbar.

Die Erfindung ist jedoch nicht auf die vorstehend beschriebenen Ausführungsbeispiele beschränkt, sondern kann auch in anderen Anwendungskontexten implementiert sein. So ist es möglich, dass die Schritte des Verfahrens nicht alle auf derselben computer-basierten Einheit ausgeführt werden, sondern auf unterschiedlichen Einheiten, wie beispielsweise auf dem Gerät G, auf der Registry 10 und/oder auf der Instanziierungseinheit I. Teilweise kann auch die Reihenfolge der Schritte modifiziert werden. Ebenso ist es möglich, das vorstehend beschriebene Authentisierungssystem noch zu erweitern, und Identitätsüberprüfungen seitens des Gerätes G bereitzustellen, so dass die Identität des jeweiligen Patienten auch lokal am Gerät G überprüft werden kann (beispielsweise durch optische Bildkontrolle, Überprüfung von biometrischen Daten oder Bestätigung von demographischen Daten etc.). Diese Ausführungsform ist besonders wertvoll in medizinischen Notfallsituationen, in denen der Patient möglicherweise nicht mehr bei Bewusstsein ist und so eine Hilfsperson in die Lage gesetzt wird, den Authentisierungsvorgang für den Patienten zu übernehmen, und diesen lokal zu authentisieren, um Notfallrelevante Datensätze vom Repository 12 abzufragen (beispielsweise Dauerdiagnosen, Unverträglichkeiten oder Allergien etc.). Andere Abweichungen liegen ebenfalls im Rahmen der Erfindung, so dass sich der Schutzbereich allein durch die nachfolgend aufgeführten Patentansprüche bestimmt.

## Patentansprüche

1. Authentisierungssystem zur Authentisierung jeweils eines mobilen, elektronischen Gerätes (G) aus einer Menge von mobilen Geräten (G) gegenüber einem zentralen Server zum gesicherten Datenaustausch von medizinischen Daten zwischen Gerät (G) und Server, wobei der Server auf ein Repository (12) mit den medizinischen Daten zugreifen kann, umfassend:
- Eine zentrale Instanziierungseinheit (I), die zur Instanziierung des jeweiligen Gerätes (G) bestimmt ist, indem die Instanziierungseinheit (I) jeweils eine individualisierte Geräte-spezifische Applikation als Verschlüsselungseinheit (V) auf dem jeweiligen Gerät (G) installiert und wobei die Applikation einen Schlüssel (40) und eine Geräte-Kennung (50) in versteckter Form in einem Programmspeicher (30) des Gerätes (G) ablegt, wobei die Instanziierungseinheit (I) eine Zuordnung zwischen Geräte-Kennung (50) und Schlüssel (40) in einem zentralen, geschützten Speicher ablegt
- Eine Verschlüsselungseinheit (V), die lokal auf dem Gerät (G) installiert ist und zum Erzeugen einer digitalen Signatur (SIG) bestimmt ist, wobei die Signatur (SIG) mit dem von der Instanziierungseinheit (I) hinterlegten Schlüssel (40) verschlüsselt ist und erzeugt wird aus einem Signatur-Urbild (SIG-UB), zumindest umfassend die Geräte-Kennung (50) und einen Zeitstempel (60), und wobei die Verschlüsselungseinheit (V) weiterhin zum Versenden zumindest der Signatur (SIG), sowie der Geräte-Kennung (50) an den Server bestimmt ist
- Eine Entschlüsselungseinheit (E), die auf dem zentralen Server installiert ist und wobei die Entschlüsselungseinheit (E) ein Zugriffsmodul (Z) auf den zentralen, geschützten Speicher umfasst,
wobei die Entschlüsselungseinheit (E) dazu bestimmt ist, die vom Gerät (G) gesendete Signatur (SIG) mit der Geräte-Kennung (50) zu empfangen und aus der Geräte-Kennung (50) unter Zugriff auf den zentralen, geschützten Speieher den jeweils zugeordneten Schlüssel (40') zur Entschlüsselung auszulesen, um mit dem Schlüssel (40') die empfangene Signatur (SIG) zu entschlüsseln und vom Signatur-Urbild (SIG-UB) als Entschlüsselungsergebnis die Geräte-Kennung (50) auszulesen, wobei die Entschlüsselungseinheit (E) weiter dazu bestimmt ist, das Entschlüsselungsergebnis mit der Geräte-Kennung (50) auf Übereinstimmung zu vergleichen und bei Übereinstimmung ist die Entschlüsselungseinheit (E) weiter dazu bestimmt, mit der ausgelesenen Geräte-Kennung (50) einen Zugriff auf das Repository (12) auszuführen.

2. Authentisierungssystem nach Anspruch 1, bei dem das Signatur-Urbild (SIG-UB) einen Geräte-bezogenen Identifikationsdatensatz umfasst.

3. Authentisierungssystem nach einem der vorstehenden Ansprüche, bei dem das Signatur-Urbild (SIG-UB) einen Anwender-bezogenen Identifikationsdatensatz umfasst.

4. Authentisierungssystem nach einem der vorstehenden Ansprüche, bei dem das Signatur-Urbild (SIG-UB) invariante Geräte-spezifische und/oder Anwender-spezifische Authentisierungsdatensätze (51), eine Zufallszahl und/oder einen Zeitstempel (60) umfasst, die sowohl der Verschlüsselungseinheit (V) als auch der Entschlüsselungseinheit (E) bekannt sind.

5. Authentisierungssystem nach einem der vorstehenden Ansprüche, bei dem die Instanziierungseinheit (I) einen privaten Schlüssel (40) in dem Gerät (G) geschützt hinterlegt und bei dem auf dem Server (10) ein öffentlicher Schlüssel (40') für jedes Gerät (G) und/oder jede Geräte-Kennung (50) hinterlegt ist.

6. Authentisierungssystem nach einem der vorstehenden Ansprüche, bei dem die Instanziierungseinheit (I) auf dem Server (10) ausgebildet ist.

7. Authentisierungssystem nach einem der vorstehenden Ansprüche, bei dem das Gerät (G) ein mobiles computergestütztes elektronisches Gerät mit programmierbarer Ausführungseinheit ist, insbesondere ein Smartphone.

8. Authentisierungssystem nach einem der vorstehenden Ansprüche, bei dem die Verschlüsselungseinheit (V) als zur Programmierung des mobilen Gerätes (G) vorbereitete Applikation ausgebildet ist und bei dem die Verschlüsselungseinheit (V) die Signatur (SIG) mit der Geräte-Kennung (50) über einen standardisierten, klartextidentifizierbaren Kommunikationskanal an den Server (10) überträgt.

9. Authentisierungssystem nach einem der vorstehenden Ansprüche, bei dem eine Zeitspanne vorkonfigurierbar ist, innerhalb derer der Authentisierungsprozess abgeschlossen sein muss und bei dem bei Überschreiten der Zeitspanne eine Fehlermeldung ausgegeben wird und der Authentisierungsprozess erneut gestartet werden kann oder eine Neu-Installation erforderlich wird.

10. Authentisierungssystem nach einem der vorstehenden Ansprüche, bei dem automatisch erfasst wird, ob der Schlüssel (40) und/oder die Geräte-Kennung (50) korrumpiert ist und/oder ob eine vorkonfigurierte Sicherheitszeitspanne überschritten ist, um bedarfsweise eine Neu-Installation der Verschlüsselungseinheit (V) auf dem Gerät (G) zu veranlassen.

11. Authentisierungssystem nach einem der vorstehenden Ansprüche, bei dem der Datenaustausch zwischen Gerät (G) und Server (10) einen Daten-Upload und/oder einen Daten-Download umfasst.

12. Instanziierungseinheit (I) mit einer Entschlüsselungseinheit (E), **dadurch gekennzeichnet, dass** die Instanziierungseinheit (I) zum Einsatz in einem Authentisierungssystem nach einem der vorstehenden Systemansprüche bestimmt ist, und die auf dem zentralen Server (10) installiert ist und wobei die Entschlüsselungseinheit (E) ein Zugriffsmodul (Z) auf den Speicher (12) umfasst, wobei die Entschlüsselungseinheit (E) dazu bestimmt ist, die vom Gerät (G) gesendete Signatur (SIG) mit der Geräte-Kennung (50) zu empfangen und aus der Geräte-Kennung (50) unter Zugriff auf den zentralen, geschützten Speicher den Schlüssel (40') zur Entschlüsselung auszulesen, um mit dem Schlüssel (40') die empfangene Signatur (SIG) zu entschlüsseln und vom resultierenden Signatur-Urbild (SIG-UB) als Entschlüsselungsergebnis die Geräte-Kennung (50) auszulesen, wobei die Entschlüsselungseinheit (E) weiter dazu bestimmt ist, das Entschlüsselungsergebnis mit der Geräte-Kennung (50) auf Übereinstimmung zu vergleichen und bei Übereinstimmung ist die Entschlüsselungseinheit (E) weiter dazu bestimmt, mit der ausgelesenen Geräte-Kennung (50) einen Zugriff auf das Repository (12) auszuführen.

13. Verfahren zur Authentisierung jeweils eines mobilen, elektronischen Gerätes (G) aus einer Menge von mobilen Geräten (G) gegenüber einem zentralen Server zum gesicherten Datenaustausch von medizinischen Daten zwischen Gerät (G) und Server, wobei der Server auf ein Repository (12) mit den medizinischen Daten zugreifen kann, umfassend folgende Verfahrensschritte:
- Instanziieren (A) des jeweiligen Gerätes (G), indem jeweils eine individualisierte Geräte-spezifische Applikation als Verschlüsselungseinheit (V) auf dem jeweiligen Gerät (G) installiert wird und wobei ein Schlüssel (40) und eine Geräte-Kennung (50) in versteckter Form in einem Programmspeicher (30) des Gerätes (G) gespeichert werden, wobei eine Zuordnung zwischen Geräte-Kennung (50) und Schlüssel (40) in einem zentralen geschützten Speicher (MEM) ablegt wird
- Erzeugen (D) einer digitalen Signatur (SIG) lokal auf dem Gerät (G), wobei die Signatur (SIG) mit dem von der Instanziierungseinheit (I) hinterlegten Schlüssel (40) verschlüsselt ist und erzeugt wird aus einem Signatur-Urbild (SIG-UB) zumindest umfassend: Die Geräte-Kennung (50) und einem Zeitstempel (60),
- Versenden (D1) zumindest der Signatur (SIG) mit der Geräte-Kennung (50) an den Server (10)
- Empfangen (F) der vom Gerät (G) gesendete Signatur (SIG) mit der Geräte-Kennung (50) auf dem zentralen Server (10)
- Erfassen (H) der Geräte-Kennung (50)
- Zugreifen (K) auf den zentralen, geschützten Speicher (MEM) mit der erfassten Geräte-Kennung (50), um den Schlüssel (40') zur Entschlüsselung auszulesen
- Entschlüsseln (L) der empfangenen Signatur (SIG) mit dem Schlüssel (40') und Erzeugen eines Entschlüsselungsergebnisses (SIG-UB), umfassend eine ausgelesene Geräte-Kennung (50)
- Vergleich (M) des Entschlüsselungsergebnisses mit der Geräte-Kennung (50) auf Übereinstimmung
- Bei Übereinstimmung: Zugriff (N) mit der ausgelesenen Geräte-Kennung (50) auf das Repository (12).

14. Computerprogrammprodukt, wobei das Computerprogrammprodukt ein Computerprogramm umfasst, das auf einem Datenträger oder auf einem Speicher eines Computers gespeichert ist und das von dem Computer lesbare Befehle umfasst, die zur Ausführung des Verfahrens nach dem vorstehenden Verfahrensanspruch bestimmt ist, wenn die Befehle auf dem Computer ausgeführt werden.

## Claims

1. Authentication system for the authentication of a particular mobile electronic device (G) from a plurality of mobile devices (G) against a central server for the secure exchange of medical data between device (G) and server, wherein the server is able to access a repository (12) containing the medical data, comprising:
- A central instantiating unit (I) which is intended for instantiating the particular device (G), where in each case the instantiating unit (I) installs an individualized device-specific application as an encryption unit (V) on the particular device (G) and wherein the application stores a key (40) and a device ID (50) in hidden form in a program memory (30) of the device (G), wherein the instantiating unit (I) stores an association between device ID (50) and key (40) in a central protected memory
- An encryption unit (V) which is installed locally on the device (G) and is intended for generating a digital signature (SIG), wherein the signature (SIG) is encrypted using the key (40) stored by the instantiating unit (I) and is generated from a signature prototype (SIG-UB), comprising at least the device ID (50) and a time stamp (60), and wherein the encryption unit (V) is furthermore intended for sending at least the signature (SIG) and also the device ID (50) to the server
- A decryption unit (E) which is installed on the central server and wherein the decryption unit (E) comprises an access module (Z) to the central protected memory, wherein the decryption unit (E) is intended for receiving the signature (SIG) sent by the device (G) with the device ID (50) and for reading out the particular associated key (40') for decryption from the device ID (50) by accessing the central protected memory in order to decrypt the received signature (SIG) using the key (40') and from the signature prototype (SIG-UB) to read out the device ID (50) as the decryption result, wherein the decryption unit (E) is further intended for comparing the decryption result with the device ID (50) for a match and when a match is found the decryption unit (E) is further intended for executing an access to the repository (12) using the device ID (50) which has been read out.

2. Authentication system according to claim 1, wherein the signature prototype (SIG-UB) comprises a device-related identification data record.

3. Authentication system according to one of the preceding claims, wherein the signature prototype (SIG-UB) comprises a user-related identification data record.

4. Authentication system according to one of the preceding claims, wherein the signature prototype (SIG-UB) comprises invariant device-specific and/or user-specific authentication data records (51), a random number and/or a time stamp (60), which are known both to the encryption unit (V) and also to the decryption unit (E).

5. Authentication system according to one of the preceding claims, wherein the instantiating unit (I) stores a private key (40) in protected fashion in the device (G) and wherein a public key (40') is stored on the server (10) for each device (G) and/or each device ID (50).

6. Authentication system according to one of the preceding claims, wherein the instantiating unit (I) is implemented on the server (10).

7. Authentication system according to one of the preceding claims, wherein the device (G) is a mobile computer-based electronic device with a programmable execution unit, in particular a smartphone.

8. Authentication system according to one of the preceding claims, wherein the encryption unit (V) is implemented as an application prepared for programming the mobile device (G) and wherein the encryption unit (V) transmits the signature (SIG) with the device ID (50) over a standardised communication channel identifiable in plain text to the server (10).

9. Authentication system according to one of the preceding claims, wherein a time span can be preconfigured within which the authentication process must have been completed and wherein if the time span is exceeded an error report is output and the authentication process can be started again or a reinstallation becomes necessary.

10. Authentication system according to one of the preceding claims, wherein it is automatically detected whether the key (40) and/or the device ID (50) is corrupted and/or whether a preconfigured security time span is exceeded, in order where necessary to initiate a reinstallation of the encryption unit (V) on the device (G).

11. Authentication system according to one of the preceding claims, wherein the data exchange between device (G) and server (10) comprises a data upload and/or a data download.

12. Instantiating unit (I) with a decryption unit (E), **characterised in that** the instantiating unit (I) is intended for use in an authentication system according to one of the preceding system claims, and which is installed on the central server (10) and wherein the decryption unit (E) comprises an access module (Z) to the memory (12), wherein the decryption unit (E) is intended for receiving the signature (SIG) sent by the device (G) with the device ID (50) and for reading out the key (40') for decryption from the device ID (50) by accessing the central protected memory in order to decrypt the received signature (SIG) using the key (40') and from the resulting signature prototype (SIG-UB) to read out the device ID (50) as the decryption result, wherein the decryption unit (E) is further intended for comparing the decryption result with the device ID (50) for a match and when a match is found the decryption unit (E) is further intended for executing an access to the repository (12) using the device ID (50) which has been read out.

13. Method for the authentication of a particular mobile electronic device (G) from a plurality of mobile devices (G) against a central server for the secure exchange of medical data between device (G) and server, wherein the server is able to access a repository (12) containing the medical data, comprising the following method steps:
- Instantiation (A) of the particular device (G), where in each case an individualized device-specific application is installed as an encryption unit (V) on the particular device (G) and wherein a key (40) and a device ID (50) are stored in hidden form in a program memory (30) of the device (G), wherein an association between device ID (50) and key (40) is stored in a central protected memory (MEM)
- Generation (D) of a digital signature (SIG) locally on the device (G), wherein the signature (SIG) is encrypted with the key (40) stored by the instantiating unit (I) and is generated from a signature prototype (SIG-UB) comprising at least: The device ID (50) and a time stamp (60),
- Sending (D1) of at least the signature (SIG) with the device ID (50) to the server (10)
- Receipt (F) of the signature (SIG) with the device ID (50) sent by the device (G) on the central server (10)
- Detection (H) of the device ID (50)
- Access (K) to the central protected memory (MEM) with the detected device ID (50) in order to read out the key (40') for the decryption
- Decryption (L) of the received signature (SIG) using the key (40') and generation of a decryption result (SIG-UB), comprising a device ID (50) which has been read out
- Comparison (M) of the decryption result with the device ID (50) for a match
- When a match is found: Access (N) using the device ID (50) which has been read out to the repository (12).

14. Computer program product, wherein the computer program product comprises a computer program which is stored on a data medium or in a memory of a computer and comprises commands readable by the computer, intended for carrying out the method according to the preceding method claim when the commands are executed on the computer.

## Revendications

1. Système d'authentification pour authentifier respectivement un appareil (G) électronique mobile parmi un ensemble d'appareils (G) mobiles, vis-à-vis d'un serveur central pour l'échange sécurisé de données médicales entre appareil (G) et serveur, le serveur pouvant accéder à un dépôt (12) ayant les données médicales, comprenant :
- une unité (I) centrale d'instanciation, qui est destinée à instancier l'appareil (G) respectif, par le fait que l'unité (I) d'instanciation installe respectivement une application individualisée spécifique à l'appareil, comme unité (V) de chiffrement sur l'appareil (G) respectif et dans lequel l'application mémorise une clé (40) et une caractérisation (50) d'appareil, sous forme cachée, dans une mémoire (30) de programme de l'appareil (G), l'unité (I) d'instanciation mémorisant une affectation entre caractérisation (50) d'appareil et clé (40) dans une mémoire centrale protégée,
- une unité (V) de chiffrement, qui est installée localement sur l'appareil (G) et qui est destinée à produire une signature (SIG) numérique, la signature (SIG) étant chiffrée par la clé (40), mémorisée par l'unité (I) d'instanciation, et étant produite à partir d'un original (SIG-UB) de signature, comprenant au moins la caractérisation (50) d'appareil et un horodatage (60), et dans lequel l'unité (V) de chiffrement est destinée, en outre, à envoyer au moins la signature (SIG), ainsi que la caractérisation (50) d'appareil, au serveur,
- une unité (E) de déchiffrement, qui est installée sur le serveur central et dans lequel l'unité (E) de déchiffrement comprend un module (Z) d'accès à la mémoire centrale protégée,
dans lequel l'unité (E) de déchiffrement est destinée à recevoir la signature (SIG), envoyée par l'appareil (G), avec la caractérisation (50) d'appareil et à lire, à partir de la caractérisation (50) d'appareil, en ayant accès à la mémoire centrale protégée, la clé (40') associée respectivement pour le déchiffrement, afin de déchiffrer, par la clé (40'), la signature (SIG) reçue et de lire de l'original (SIG-UB) de signature, comme résultat de déchiffrement, la caractérisation (50) d'appareil, l'unité (E) de déchiffrement étant destinée, en outre à comparer le résultat de déchiffrement à la caractérisation (50) d'appareil pour voir s'ils coïncident et, s'il y a coïncidence, l'unité (E) de déchiffrement est destinée, en outre, à exécuter, par la caractérisation (50) d'appareil qui a été lue, un accès au dépôt (12).

2. Système d'authentification suivant la revendication 1, dans lequel l'original (SIG-UB) de signature comprend un jeu de données d'identification se rapportant à un appareil.

3. Système d'authentification suivant l'une des revendications précédentes, dans lequel l'original (SIG-UB) de signature comprend un jeu de données d'identification se rapportant à un utilisateur.

4. Système d'authentification suivant l'une des revendications précédentes, dans lequel l'original (SIG-UB) de signature comprend des jeux (51) de données d'authentification invariants spécifiques à un appareil et/ou spécifiques à un utilisateur, un nombre aléatoire et/ou un horodatage (60), qui sont connus à la fois de l'unité (V) de chiffrement et de l'unité (E) de déchiffrement.

5. Système d'authentification suivant l'une des revendications précédentes, dans lequel l'unité (I) d'instanciation mémorise, de manière protégée, une clé (40) privée dans l'appareil (G) et dans lequel il est mémorisé, sur le serveur (10), une clé (40') publique pour chaque appareil (G) et/ou chaque caractérisation (50) d'appareil.

6. Système d'authentification suivant l'une des revendications précédentes, dans lequel l'unité (I) d'instanciation est constituée sur le serveur (10).

7. Système d'authentification suivant l'une des revendications précédentes, dans lequel l'appareil (G) est un appareil électronique mobile assisté par ordinateur, ayant une unité d'exécution programmable, en étant notamment un smartphone.

8. Système d'authentification suivant l'une des revendications précédentes, dans lequel l'unité (V) de chiffrement est constituée sous la forme d'une application préparée pour la programmation de l'appareil (G) mobile et dans lequel l'unité (V) de chiffrement transmet au serveur (10) la signature (SIG) avec la caractérisation (50) d'appareil par un canal de communication normalisé et pouvant être identifié en texte en clair.

9. Système d'authentification suivant l'une des revendications précédentes, dans lequel il peut être configuré à l'avance un laps de temps, dans lequel le processus d'authentification doit être terminé et dans lequel, si le laps de temps est dépassé, il est émis un message d'erreur et le processus d'authentification peut être recommencé ou une installation nouvelle être nécessaire.

10. Système d'authentification suivant l'une des revendications précédentes, dans lequel il est détecté automatiquement si la clé (40) et/ou la caractérisation (50) d'appareil est corrompue et/ou si un laps de temps de sécurité configuré à l'avance est dépassé, pour provoquer, en cas de besoin, une nouvelle installation de l'unité (V) de chiffrement sur l'appareil (G).

11. Système d'authentification suivant l'une des revendications précédentes, dans lequel l'échange de données entre l'appareil (G) et le serveur (10) comprend un upload de données et/ou un download de données.

12. Unité (I) d'instanciation, ayant une unité (E) de déchiffrement, **caractérisée en ce que**
l'unité (I) d'instanciation est destinée à être utilisée dans un système d'authentification suivant l'une des revendications de système précédentes et qui est installée sur le serveur (10) central et dans laquelle l'unité (E) de déchiffrement comprend un module (Z) d'accès à la mémoire (12), l'unité (E) de déchiffrement étant destinée à recevoir la signature (SIG), envoyée par l'appareil (G), avec la caractérisation (50) d'appareil et à lire pour le déchiffrement la clé (40') à partir de la caractérisation (50) d'appareil, en ayant accès à la mémoire centrale protégée, et à déchiffrer, par la clé (40') la signature (SIG) reçue et à lire la caractérisation (50) d'appareil, comme résultat du déchiffrement, à partir de l'original (SIG-UB) de signature résultant, l'unité (E) de déchiffrement étant destinée, en outre, à comparer le résultat du déchiffrement à la caractérisation (50) d'appareil, pour voir s'il y a coïncidence, et s'il y a coïncidence, l'unité (E) de déchiffrement est destinée, en outre, à réaliser, par la caractérisation (50) d'appareil qui a été lue, un accès au dépôt (12).

13. Procédé d'authentification respectivement d'un appareil (G) électronique mobile parmi un ensemble d'appareils (G) mobiles vis-à-vis d'un serveur central pour l'échange sécurisé de données médicales entre appareil (G) et serveur, le serveur pouvant accéder à un dépôt (12) ayant les données médicales, comprenant les stades de procédé suivants :
- instanciation (A) de l'appareil (G) respectif, en installant sur l'appareil (G) respectif respectivement une application individualisée spécifique à l'appareil, comme unité (V) de chiffrement, et dans lequel on mémorise, dans une mémoire (30) de programme de l'appareil (G) sous forme cachée, une clé (40) et une caractérisation (50) d'appareil, dans lequel on mémorise, dans une mémoire (MEM) centrale protégée, une affectation entre caractérisation (50) d'appareil et clé (40),
- production (D) d'une signature (SIG) numérique localement sur l'appareil (G), la signature (SIG) étant chiffrée par la clé (40) mémorisée par l'unité (I) d'instanciation et produite à partir d'un original (SIG-UB) de signature, comprenant au moins : la caractérisation (50) d'appareil et un horodatage (60),
- envoi (D1) au serveur (10) au moins de la signature (SIG) avec la caractérisation (50) d'appareil,
- réception (F) de la signature (SIG), envoyée par l'appareil (G), avec la caractérisation (50) d'appareil, au serveur (10) central,
- détection (H) de la caractérisation (50) d'appareil,
- accès (K) à la mémoire (MEM) centrale protégée, par la caractérisation (50) d'appareil détectée, pour lire la clé (40') de déchiffrement,
- déchiffrement (L) de la signature (SIG) reçue par la clé (40') et production d'un résultat (SIG-UB) de déchiffrement, comprenant une caractérisation (50) d'appareil lue,
- comparaison (M) du résultat de déchiffrement à la caractérisation (50) d'appareil pour voir s'il y a coïncidence,
- s'il y a coïncidence : accès (N) par la caractérisation (50) d'appareil lue au dépôt (12).

14. Produit de programme d'ordinateur, dans lequel le produit de programme d'ordinateur comprend un programme d'ordinateur, qui est mémorisé sur un support de données ou dans une mémoire d'un ordinateur et qui comprend des instructions pouvant être déchiffrées par l'ordinateur, qui est destiné à l'exécution du procédé suivant la revendication de procédé précédente lorsque les instructions sont réalisées sur l'ordinateur.
